# EUROPÄISCHE PATENTSCHRIFT

(11) **EP 0 610 246 B1**
(45) Veröffentlichungstag und Bekanntmachung des Hinweises auf die Patenterteilung: **10.12.1997**
(21) Anmeldenummer: 92920588.8
(22) Anmeldetag: 23.09.1992
(51) Int. Cl.: C12N 15/15, C07K 14/81, A61K 38/55

(54) **NEUES THROMBININHIBITORISCHES PROTEIN AUS ZECKEN**
NOVEL THROMBIN-INHIBITING PROTEIN FROM TICKS
NOUVELLE PROTEINE INHIBITRICE DE LA TROMBINE ISOLEE DANS LES TIQUES

(30) Priorität: 31.10.1991 DE 4136087
(43) Veröffentlichungstag der Anmeldung: 17.08.1994
(73) Patentinhaber: BASF Aktiengesellschaft, 67063 Ludwigshafen (DE)
(72) Erfinder: FRIEDRICH, Thomas, D-6100 Darmstadt (DE); KOERWER, Wolfgang, D-6718 Gruenstadt (DE); KROEGER, Burkhard, D-67117 Limburgerhof (DE); BIALOJAN, Siegfried, D-6836 Oftersheim (DE)
(86) Internationale Anmeldenummer: EP9202198
(87) Internationale Veröffentlichungsnummer: WO9309231

(56) Entgegenhaltungen:
- WO-A-91/04275
- JOURNAL OF BIOLOGICAL CHEMISTRY. (MICROFILMS) Bd. 265, Nr. 29, 15. Oktober 1990, BALTIMORE, MD US Seiten 17746 - 17752 M. NEEPER ET AL. 'Characterization of Recombinant Tick Anticoagulant Peptide'

## Beschreibung

Die vorliegende Erfindung betrifft ein neues thrombininhibitorisches Protein aus Zecken sowie Verfahren zu dessen Herstellung.

Thrombin-Inhibitoren sind wichtige therapeutische Substanzen, die beispielsweise zur Prophylaxe oder Behandlung von Thrombosen oder arteriellen Reokklusionen verwendet werden.

In der deutschen Offenlegungsschrift DE 39 31 839 wird ein Thrombinininhibitor, der aus der Lederzecke Ornithodoros moubata isoliert worden ist, beschrieben. Dieses Protein besitzt ein Molekulargewicht von etwa 15000 Dalton, einen isoelektrischen Punkt von pH 4-5 und die N-terminale Aminosäuresequenz SDYEFPPPKKXRPG.

In der europäischen Offenlegungsschrift EP 345 614 wird der thrombininhibitorische Wirkstoff Amblyommin beschrieben, der aus Schildzecken isoliert wird. Es handelt sich dabei um ein Protein mit einem Molekulargewicht von 20 000 - 30 000 Dalton und einem isoelektrischen Punkt zwischen 5,05 und 5,65.

Bisher wurde jedoch noch kein Protein mit thrombininhibitorischer Wirkung gefunden, das als Arzneimittel hinsichtlich hoher Wirksamkeit, fehlender Antigenizität, langer biologischer Halbwertzeit, geringer Nebenwirkung wie z.B. Blutungsneigung, vorteilhaft geeignet ist.

Der Erfindung lag daher die Aufgabe zugrunde, neue Thrombin-Inhibitoren zur Verfügung zu stellen, die als Arzneimittel hinsichtlich der oben genannten Eigenschaften geeignet sind.

Demgemäß wurde ein neues thrombininhibitorisches Protein aus Zecken isoliert.

Das neue Protein besitzt folgende physikochemischen Eigenschaften. Durch Molekularsiebchromatographie wird ihm ein Molekulargewicht von 26000 - 29000 Dalton zugeordnet. Durch Elektrophorese in einem Tricine SDS-Polyacrylamidgel wird ein Molekulargewicht von 11000 ± 1500 Dalton bestimmt. Durch Elektrophorese in einem SDS-Polyacrylamidgel nach Lämmli wird ein Molekulargewicht von 25700 ± 3000 Dalton bestimmt. Die Bestimmung des isoelektrischen Punktes ergibt einen pH-Wert zwischen 4 und 5.

Das Protein bindet spezifisch an eine Thrombin-Affinitätssäule. Es hemmt die biologische Aktivität von Thrombin in einem in-vitro Enzymtest.

In einem Polyacrylamidgel läßt sich die Proteinbande durch Silber färbung nicht anfärben; sie ist nur als nicht-gefärbter Fleck auf einem gefärbten Hintergrund sichtbar.

Der Aminoterminus des Proteins ist blockiert. Folgende Aminosäureteilsequenzen wurden von dem Protein bestimmt:

Sequenz I:   Val-Ala-Lys-Phe-Pro-Ala-(Ala)-Asn-Ser-Gly-Ser-
             Glu-Thr-Gly

Sequenz II:  His-Ala-(Cys)-Phe-Glu

Sequenz III: 90 % Arg-Val-Ser-Asp-Phe-Glu
             10 % Phe-Ala-(Glu/His)-Lys

Sequenz IV:  70 % Phe-Val-Tyr-Thr-Ile-Glu
             30 % Ala-Phe-Gln-Gly

Die in Klammern angegebenen Aminosäuren sind nicht völlig sicher identifiziert. Bei Sequenz III und IV handelt es sich jeweils um Gemische zweier Sequenzen, die in der angegebenen Intensität auftraten.

Das neue Protein läßt sich aus Zecken der Gattung Ornithodoros isolieren. Hierzu werden die Zecken zweckmäßigerweise in einem Puffer bei pH 6 bis 9, vorzugsweise pH 7 bis 8, aufgenommen und mit einem Homcgenisator, vorzugsweise einem Mixer, homogenisiert. Anschließend werden die unlöslichen Bestandteile abgetrennt, bevorzugt abzentrifugiert.

Aus der so erhaltenen Lösung kann das Protein weiter gereinigt werden, indem mit einem Fällungsmittel, bevorzugt Trichloressigsäure, andere Proteine aus der Lösung ausgefällt und anschließend abgetrennt werden.

Die weitere Reinigung des Proteins kann über chromatographische Methoden, bevorzugt Ionenaustauschchromatographie und/ oder Affinitätschromatographie erfolgen. Besonders bevorzugt ist ein Reinigungsschritt über Thrombin-Affinitätschromatographie.

Die Reinigung des Proteins kann über einen Thrombin-Aktivitätstest verfolgt werden. Hierzu benutzt man zweckmäßigerweise einen optischen Test, bei dem ein chromogenes Substrat, beispielsweise Chromozym T, durch Thrombin umgesetzt wird. Die das neue Protein enthaltenden Fraktionen können bei Zugabe in diesen optischen Test an ihrer Thrombin inhibierenden Wirkung erkannt werden.

Besonders geeignet zur Herstellung des erfindungsgemäßen Proteins sind gentechnische Verfahren.

Hierzu wird in an sich bekannter Weise eine cDNA-Genbank aus der Zecke angelegt. Aus dieser Genbank kann das für das erfindungsgemäße Protein kodierende Gen isoliert werden, indem man beispielsweise eine DNA-Probe herstellt, deren Sequenz von der oben beschriebenen N-terminalen Aminosäuresequenz durch Rückübersetzung gemäß dem genetischen Code erhalten wird. Durch Hybridisierung mit dieser DNA-Probe läßt sich das entsprechende Gen auffinden und isolieren.

Für die Herstellung des entsprechenden Gens oder von Teilen des Gens kann aber auch die Polymerase-Chain-Reaction (PCR)-Technik eingesetzt werden. Beispielsweise läßt sich mit Hilfe eines Primers, dessen Sequenz durch Rückübersetzung aus den oben beschriebenen Aminosäureteilsequenzen erhalten wurde, und eines zweiten Primers, dessen Sequenz komplementär zum 3'-Ende des cDNA-Genfragments ist, bevorzugt mit der Sequenz Poly(dT), das cDNA-Genfragment für das erfindungsgemäße Protein durch PCR-Technik herstellen. Das entsprechende Gen läßt sich auch isolieren, indem man eine Expressionsgenbank von Zecken anlegt und diese mit einem Antikörper, der gegen das erfindungsgemäße Protein gerichtet ist, absucht.

Nachdem das entsprechende Gen isoliert worden ist, kann es durch gentechnische Verfahren in Organismen, z.B. in Bakterien, Hefen, eukaryontischen Zellen, mit Hilfe eines Expressionsvektors in an sich bekannter Weise exprimiert werden. Aus diesen rekombinanten Wirtssystemen läßt sich das Protein aufgrund der oben beschriebenen physikochemischen Eigenschaften isolieren.

Die generelle Vorgehensweise zur gentechnischen Herstellung eines neuen Proteins bei bekannter Aminosäurepartialsequenz ist in Lehrbüchern der Gentechnologie, beispielsweise E.L. Winnacker, Gene und Klone, Verlag Chemie, Weinheim, 1984, beschrieben. Die experimentellen Bedingungen für die einzelnen Verfahren wie beispielsweise Anlegen einer Genbank, Hybridisierung, Expression eines Gens sind bei T. Manniatis, "Molecular Cloning", Cold Spring Harbor Laboratory, 1990, beschrieben.

Das erfindungsgemäße Protein wird bevorzugt in Form seiner pharmazeutisch annehmbaren Salze verwendet.

Das neue Protein besitzt blutgerinnungshemmende Eigenschaften. Es kann beispielsweise zur Prophylaxe von Thrombosen oder arteriellen Reokklusionen, zur Behandlung von Thrombosen, zur Konservierung von Blut oder bei der extrakorporalen Zirkulation verwendet werden.

Die Erfindung wird durch die folgenden Beispiele weiter veranschaulicht.

### Beispiel 1

### Reinigung des thrombininhibitorischen Proteins aus Zecken

Eine Laborkultur der Zecken (Onithodoros decoloratus) wurde bei 28°C und 80 % relativer Luftfeuchtigkeit gehalten. In 14-tägigen Abständen wurden die Zecken dadurch gefüttert, daß sie an Kaninchen saugen konnten. Zecken aller Entwicklungsstadien wurden bei -20°C eingefroren.

200 g Zecken wurden mit 1000 ml 10 mM Natriumphosphatpuffer, 150 mM NaCl (pH 7,5) homogenisiert. Das Homogenat wurde 15 Minuten bei 7000 U/min (Sorvall RC-5B, Rotor GS-3) zentrifugiert. Der Niederschlag wurde verworfen und der Überstand (1300 ml) mit 60 ml 50 gew.-%iger Trichloressigsäure durch Zutropfen über einen Zeitraum von 10 Minuten versetzt.

Die Lösung wurde mit dest. Wasser auf ein Volumen von 2000 ml aufgefüllt und 15 Minuten gerührt.

Danach wurde zentrifugiert (20 Minuten, 7000 U/min) und der Überstand mit Natronlauge neutralisiert.

Der neutralisierte Überstand wurde in Dialyseschläuche (Ausschlußvolumen 300 Da) gefüllt und mehrfach gegen das 10-fache Volumen 20 mM Natriumphosphat, 150 mM NaCl, pH 8,0 dialysiert.

Anschließend wurde die Lösung durch Ultrafiltration (Amicon YM5® Membran) auf ein Viertel eingeengt. Das Protein wurde durch das vierfache Volumen Aceton (auf -50°C gekühlt) gefällt.

Nachdem das Gemisch eine Stunde auf Trockeneis inkubiert worden war, wurde es 20 Minuten bei 7000 U/min zentrifugiert (Sorvall RC-5B, Rotor GS-3). Der Niederschlag wurde gesammelt, getrocknet und in 10 mM Natriumphosphatpuffer pH 7,5 resuspendiert (je 40 ml Puffer auf 5 g Niederschlag).

Ca. 45 ml des resuspendierten Niederschlags wurden auf eine Q-Sepharose Säule® (Pharmacia) aufgetragen (60 ml/h), die in 10 mM Natriumphosphatpuffer pH 7,5 äquilibriert worden war (Durchmesser 2,6 cm, Höhe 8 cm).

Es wurde mit 10 Säulenvolumen Äquilibrierungspuffer gewaschen.

Danach wurde ein linearer Gradient von 200 ml 10 mM Natriumphosphat (pH 7,5), nach 200 ml 10 mM Natriumphosphat (pH 7,5), 500 mM NaCl angelegt.

Aktive Fraktionen (gemessen durch Thrombininhibition) wurden gesammelt.

Das Protein wurde bei etwa 350 mM NaCl eluiert.

Die vereinigten aktiven Fraktionen wurden auf eine Affinitätssäule mit immobilisiertem Thrombin aufgetragen (Durchmesser 1,2 cm, Höhe 6,5 cm, 11,5 ml Säulenvolumen, 60 ml/h). Die Säule wurde gemäß Beispiel 3 hergestellt.

Die Säule wurde mit 10 mM Natriumphosphat pH 7,5 äquilibriert. Nach Auftragen der Proteinlösung wurde die Säule mit 10 Säulenvolumen Äquilibrierungspuffer gewaschen, bis die Absorption bei 280 nm auf Null zurückging.

Danach wurde mit 0,5 M NaCl, 10 mM Natriumphosphatpuffer pH 7,5 gewaschen. Unspezifisch adsorbiertes Material wurde so entfernt.

Spezifisch an Thrombin gebundenes Protein wurde mit 0,1 M Glycin, 0,5 M NaCl pH 2,8 eluiert. Die Säule wurde anschließend sofort wieder mittels Phosphatpuffer auf pH 7,5 eingestellt.

Die einzelnen Fraktionen wurden mit 0,1 M NaOH neutralisiert und auf ihre inhibitorische Wirkung gegen Thrombin untersucht.

Die durch Glycin/NaCl-Puffer pH 2,8 eluierten Fraktionen besaßen thrombininhibierende Wirkung.

Die gesammelten aktiven Fraktionen wurden, nachdem sie neutralisiert worden waren, mit Wasser verdünnt (1:10) und auf eine Mono-Q-Säule® gegeben (Pharmacia, 1 ml Säulenvolumen).

Die Säule wurde äquilibriert mit 10 mM Natriumphosphatpuffer pH 7,5 (Puffer A). Es wurde mit Puffer A gewaschen bis die Absorption auf Null zurückging (10 Minuten). Dann wurde innerhalb von 50 Minuten auf 10 mM Natriumphosphat, pH 7,5, 500 mM NaCl (Puffer B) gewechselt (Fluß 0,5 ml/min).

Die thrombininhibierende Aktivität wurde bei ca. 70 % Puffer B eluiert.

Thrombin hemmende Fraktionen wurden gesammelt.

Die gesammelten Fraktionen wurden an einer RP 318® (Biorad) HPLC-Chromatographiesäule weiter gereinigt. Die Säule wurde äquilibriert mit 0,1 gew.-%iger Trifluoressigsäure (TFA) in dest. Wasser. Die vereinicten aktiven Fraktionen wurden auf die Säule aufgetragen. Mit einem Gradienten nach 0,1 gew.-%iger TFA in Acetonitril während einer Stunde und einer Flußrate von 1 ml/min wurde die Säule eluiert. Die Absorption wurde bei 280 nm bestimmt. Es wurden 0,5 ml Fraktionen gesammelt. Die gesammelten Fraktionen wurden zur Trockene einkonzentriert und in Phosphat gepufferter Salzlösung (PBS) (0,8 g/l NaCl; 0,2 g/l HCl; 0,144 g/l Natriumphosphat; 0,2 g/l Kaliumphosphat, pH 7,5) aufgenommen sowie die inhibitorische Aktivität bestimmt.

Die Proteinbestimmung erfolgte nach der Vorschrift von Bradford (Anal. Biochem., 72, 248-254 (1976)). Als Standardprotein diente Rinderserumalbumin (Boehringer Mannheim).

Die Reinigung des thrombininhibierenden Proteins ist in der Tabelle zusammengefaßt. Die Tabelle enthält die Daten von drei voneinander unabhängigen Experimenten zur Reinigung des thrombininhibierenden Proteins.

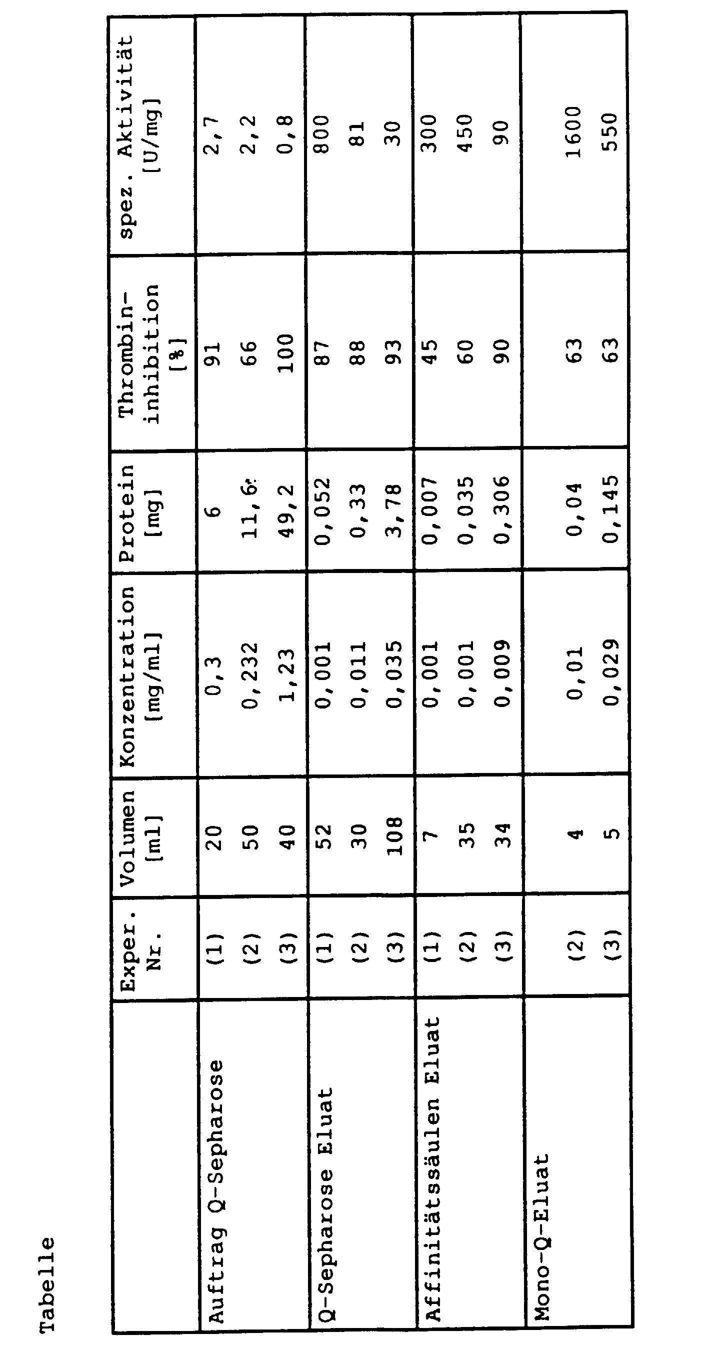

### Beispiel 2

### Bestimmung der Hemmung von Thrombin durch den Inhibitor

Thrombin (Boehringer Mannheim) wurde zu einer Endkonzentration von (25 mU/ml) in Phosphat gepufferter Salzlösung (PBS) (0,8 g/l NaCl; 0,2 g/l HCl; 0,144 g/l Natriumphosphat; 0,2 g/l Kaliumphosphat, pH 7,5) gelöst.

Chromozym TH (Boehringer Mannheim) wurde in 20 ml H₂O/Flasche gelöst.

50 µl Thrombinlösung und 100 µl Chromozym sowie 25 µl Probe oder Puffer wurden in die Näpfe einer Mikrotiterplatte gegeben. Sofort danach wurde zur Zeit 0 und nach 30 Minuten bei 37°C die Absorption bei 405 nm gemessen.

Bei starker Eigenfarbe der Probe wurde eine weitere Kontrolle ohne Thrombin wie oben behandelt.

Durch die Aktivität des Thrombins wird aus dem chromogenen Farbsubstrat ein bei 405 nm absorbierender Farbstoff freigesetzt. Die Hemmung des Thrombins durch einen Thrombininhibitor ist an einer geringeren Absorptionszunahme bei 405 nm erkennbar und wurde mit Hilfe einer Eichkurve quantifieziert.

### Beispiel 3

### Herstellung einer Affinitätssäule mit Thrombin als Ligand

### a) Kopplung:

2 g CNBr aktivierte Sepharose (Pharmacia) wurden mit 200 ml 1 mM HCl auf einer Nutsche gewaschen. Das Gel wurde in 100 mM NaHCO₃, 500 mM NaCl pH 8,3 aufgenommen und sofort mit 10000 Units Thrombin (Sigma) in 100 mM NaHCO₃, 500 mM NaCl, pH 8,3 gemischt.

Die Lösung wurde 24 Stunden bei 4°C vorsichtig geschüttelt.

### b) Blockierung:

Das Gelmaterial wurde nach Absetzen mit 100 mM NaHCO₃, 500 mM NaCl, pH 8,3 gewaschen. Die Sepharose wurde dann mit 100 mM NaHCO₃, 500 mM NaCl, 1 M Ethanolamin pH 8,3 für 2 Stunden inkubiert.

### c) Vorbereitung:

Zur Entfernung des ungebundenen Thrombins wurde das Gelmaterial vor Gebrauch noch einmal in der Säule mit 20 Säulenvolumen PBS pH 7,4 gewaschen.

### Beispiel 4

### Bestimmung des Molekulargewichts durch Molekularsieb- chromatographie.

Bei einer Flußgeschwindigkeit von 1 ml/min wurde durch MonoQ®-Chromatographie gereinigtes Material in 20 mM Natriumphosphat, 150 mM NaCl, pH 7,5 auf einer Molekularsiebsäule des Typs TSK® (Pharmacia, Spherogel TSK 3000® SW, 7,5 mm Durchmesser, 60 cm Höhe) getrennt.

Ebenso wurde mit den Eichproteinen verfahren (Serumalbumin MW 67000 Da, Ovalbumin MW 45000 Da, Chymotypsionogen A MW 25000 Da).

Der Logarithmus des Molekulargewichts der Eichproteine wurde gegen deren Elutionszeit in einem Diagramm aufgetragen.

In den fraktionierten Eluaten der Probe wurde die Thrombininhibition bestimmt.

Die Elutionszeit des Inhibitors ergab am Schnittpunkt mit der Eichgeraden den Logarithmus des gesuchten Molekulargewichts.

Durch diese Bestimmung wurde ein Molekulargewicht zwischen 26000 und 29000 Dalton bestimmt.

### Beispiel 5

### Bestimmung des Molekulargewichts durch Tricine-SDS-Polyacrylamidgel-Elektrophorese.

(Literatur: Analytical Biochemistry, 166, 368 - 379 (1987) Tricine-Sodium Dodecyl Sulfate-Polyacrylamide Gel Electrophoresis for the Separation of Proteins in the range from 1 - 1000 kDa, Schägger, H. and von Jagow, G.)

Die Gelelektrophorese wurde entsprechend der Literaturvorschrift bei 20 mA und 1400 V, 30 Watt, durchgeführt.

Das nach dieser Methode bestimmte Molekulargewicht betrug 11000 ± 1500 Dalton.

Als Eichproteine dienten (Intaktes Myoglobin 17,2 kDa, Bromcyanpeptid Myoglobin I+II 14,6 kDa, Bromcyanpeptid Myoglobin I 8,2 kDa, Bromcyanpeptid Myoglobin II 6,4 kDa, Bromcyanpeptid Myoglobin III 2,6 kDa, Myoglobin 1-14)

### Beispiel 6

### Bestimmung des Molekulargewichts durch SDS-Gelelektrophorese

(Literatur: Nature 227, 680-685 (1970), Cleavage of structural proteins during the assembly of the head of bacteriophage T4, Lämmli, U.K.)

Die Gelelektrophorese wurde entsprechend der Literaturvorschrift durchgeführt.

Das nach dieser Methode bestimmte Molekulargewicht betrug 25700 ± 3000 Dalton.

Das Molekulargewicht wurde anhand von folgenden Eichproteinen bestimmt:

| | |
|---|---|
| Phosphorylase b (Kaninchenmuskel) | 94000 Dalton |
| Rinderserumalbumin | 67000 Dalton |
| Ovalbumin (Eiweiß) | 43000 Dalton |
| Kohlensäureanhydratase (Rindererythrozyten) | 30000 Dalton |
| Trypsin-Inhibitor (Sojabohne) | 20000 Dalton |
| α-Lactalbumin (Kuhmilch) | 14400 Dalton |

### Beispiel 7

### Partielle Sequenzbestimmung des Inhibitors

Der wie oben beschrieben gereinigte Inhibitor war N-terminal blockiert und konnte daher nicht direkt sequenziert werden. Daher wurde eine Spaltung in Peptide mittels Protease vorgenommen.

### Reduktion und Carboxymethylierung.

2,8 ml Proteinlösung (0,029 mg/ml) wurden mit 0,28 ml Puffer (1 M Tris/HCl, 0,5 M Guanidinhydrochlorid, pH 8,6, gemischt. Danach wurden 0,116 ml Dithiothreitol (DTT, 10 mg/ml) zugegeben und 10 Minuten bei 37°C inkubiert. Danach wurde nach Zugabe von 0,185 ml Jodacetamid (10 mg/ml) für 90 Minuten bei 37°C inkubiert. Die Reaktion wurde mit 0,073 ml DTT wie oben beendet.

Das so behandelte Protein wurde über Nacht mit Trypsin bei 37°C gespalten. Die Protease wurde in einem Verhältnis zwischen 1:10 bis 1:100 (Gew.-Teil Protease:Gew.-Teil Protein) eingesetzt. Die Protease wurde nach Vorschrift des Herstellers gelöst.

Das Peptidgemisch wurde durch reversed phase HPLC an RP 318® getrennt. Das Gemisch wurde auf eine Endkonzentration von 0,1 Gew.% Trifluoressigsäure (TFA) eingestellt und in einem HPLC System von Hewlett Packard (HP 1090 Liquid Chromatograph) getrennt. Mit Lösungsmittel A (0,1 gew.-%iger TFA in H₂O) wurde 5 Minuten gewaschen. Dann wurde der Anteil von Lösungsmittel B (90 Vol.-% Acetonitril, 10 Vol.-% H₂O, 0,1 Gew.% TFA) im Verlauf von 120 Minuten auf 50 % angehoben. Die Absorption des Eluats bei 214 und 280 nm wurde gemessen. Absorbierende Fraktionen wurden gesammelt. Die Peptide wurden einer Sequenzanalyse auf einem Applied Biosystems 477 A Protein Sequencer nach Vorschrift des Geräteherstellers unterworfen.

Folgende Sequenzen wurden erhalten:

Sequenz I:   Val-Ala-Lys-Phe-Pro-Ala-(Ala)-Asn-Ser-Gly-Ser-
             Glu-Thr-Gly

Sequenz II:  His-Ala-(Cys)-Phe-Glu

Sequenz III: 90 % Arg-Val-Ser-Asp-Phe-Glu
             10 % Phe-Ala-(Glu/His)-Lys

Sequenz IV:  70 % Phe-Val-Tyr-Thr-Ile-Glu
             30 % Ala-Phe-Gln-Gly

Die in Klammern angegebenen Aminosäuren sind nicht völlig sicher identifiziert. Bei Sequenz III und IV handelt es sich jeweils um Gemische zweier Sequenzen, die in der angegebenen Intensität auftraten.

### Beispiel 8

### Bestimmung des isoelektrischen Punktes durch isoelektrische Fokussierung

Die Bestimmung wurde mit einem LKB Multiphor 2117 (Horizontalsystem) und einem LKB powersupply 2103 durchgeführt. Es wurden Fertiggele eingesetzt (Pharmacia Ampholine PAGplate pH 3,5 - 9,5). Als Standarproteine wurden Amyloglycosidase pH 3,5; Soyabean Trypsin Inhibitor, pH 4,55; β-Lactoglobulin A, pH 5,2; Bovine carbonic anhydratase, pH 5,85; Human carbonic anhydratase, pH 6,55; Horse myoglobin, pH 6,85 und 7,35; Lentil Lectin, pH 8,15, 8,45, 8,65 und Trypsinogen, pH 9,3 eingesetzt.

Die Bedingungen der Fokussierung: 1500 Volt, 30 Watt. Puffer:
- Anode: 1M Phosphorsäure
- Kathode: 1M Natronlauge

Die Platten wurden 30 Minuten zur Ausbildung eines pH-Gradienten vorfokussiert. Die Proben wurden auf Filterplättchen aufgetragen, die auf dem Gel lagen. Für 30 Minuten wurde weiterfokussiert, die Filterplättchen abgenommen, und nach weiteren 30 Minuten wurde die Fokussierung beendet. Die Gele wurden sofort in 2 mm Scheiben geschnitten und in dest. Wasser überführt. Über Nacht eluierte das Protein aus den Gelscheiben. Durch einen Thrombininhibitionstest wurde die Lage des Thrombininhibitors bestimmt. Der pH-Wert kann auch direkt durch eine pH-Elektrode bestimmt werden. Hirudin, als Vergleichssubstanz hatte einen isoelektrischen Punkt von pH 3,5 und niedriger. Der neue Inhibitor hatte einen isoelektrischen Punkt zwischen pH 4 und 5.

## Patentansprüche

1. Protein mit thrombininhibitorischer Wirkung aus Zecken der Gattung Ornithodoros, dadurch gekennzeichnet, daß es einen isoelektrischen Punkt zwischen pH 4 und 5, ein Molekulargewicht von 25700 ± 3000 Dalton, bestimmt durch SDS-Polyacrylamidgelelektrophorese nach Lämmli, und die Aminosäureteilsequenzen Val-Ala-Lys-Phe-Ala-X-Asn-Ser-Gly-Ser-Glu-Thr-Gly, His-Ala-Y-Phe-Glu, Arg-Val-Ser-Asp-Phe-Glu und Phe-Val-Tyr-Thr-Ile-Glu aufweist, worin X und Y gleich oder verschieden sein können und jeweils für eine natürlich vorkommende Aminosäure stehen.

2. DNA-Sequenz, die für ein Protein gemäß Anspruch 1 codiert.

3. Expressionsvektor, der eine DNA-Sequenz gemäß Anspruch 2 enthält.

4. Verwendung eines Proteins gemäß Anspruch 1 zur Herstellung eines Arzneimittels für die Hemmung der Blutgerinnung.

## Claims

1. A protein with a thrombin-inhibitory effect from ticks of the genus Ornithodoros, which has an isoelectric point at pH 4-5, a molecular weight of 25700 ± 3000 dalton determined by SDS polyacrylamide gel electrophoresis by the Lämmli method, and the partial amino-acid sequences Val-Ala-Lys-Phe-Ala-X-Asn-Ser-Gly-Ser-Glu-Thr- Gly, His-Ala-Y-Phe-Glu, Arg-Val-Ser-Asp-Phe-Glu and Phe-Val-Tyr-Thr-Ile-Glu, where X and Y can be identical or different and each is a naturally occurring amino acide.

2. A DNA sequence which codes for a protein as claimed in claim 1.

3. An expression vector which contains a DNA sequence as claimed in claim 2.

4. The use of a protein as claimed in claim 1 for producing a drug for inhibiting blood coagulation.

## Revendications

1. Protéine à activité inhibitrice de la thrombine, provenant de tiques du genre Ornithodoros, caractérisée en ce qu'elle possède un point isoélectrique entre pH 4 et 5, un poids moléculaire de 25700 ± 3000 daltons, déterminé par électrophorèse, sur gel de DSS-polyacrylamide selon Lämmli, et les séquences partielles d'aminoacides Val-Ala-Lys-Phe-Ala-X-Asn-Ser-Gly-Ser-Glu-Thr-Gly, His-Ala-Y-Phe-Glu, Arg-Val-Ser-Asp-Phe-Glu et Phe-Val-Tyr-Thr-lle-Glu, où X et Y peuvent être identiques ou différents et représentent chacun un aminoacide naturel.

2. Séquence d'ADN qui code pour une protéine suivant la revendication 1.

3. Vecteur d'expression qui contient une séquence d'ADN suivant la revendication 2.

4. Utilisation d'une protéine suivant la revendication 1, pour la préparation d'un médicament destiné à l'inhibition de la coagulation sanguine.
